# EUROPEAN PATENT SPECIFICATION

(11) **EP 0 292 553 B1**
(45) Date of publication and mention of the grant of the patent: **05.05.1993**
(21) Application number: 88900323.2
(22) Date of filing: 04.12.1987
(51) Int. Cl.: C12Q 1/04, G01N 33/50

(54) **PROGNOSTIC METHOD**
PROGNOSTISCHES VERFAHREN
PROCEDE PRONOSTIQUE

(30) Priority: 05.12.1986 US 938426
(43) Date of publication of application: 30.11.1988
(73) Proprietor: SERONO PHARMACEUTICAL PARTNERS, Boston, MA 02109 (US)
(72) Inventor: BITRAN, J. Joint Sect. Hemat./Oncol., Chicago, IL 60615 (US); HARRINGTON-FOWLER, Linda, Medfield, MA 02052 (US)
(74) Representative: Kraus, Walter, Dr.
(86) International application number: US8703208
(87) International publication number: WO8804327

(56) References cited:
- EP-A- 0 234 973
- Biological Abstracts, vol. 80, August 1985, (Philadelphia, PA, US) J.S. McDOUGAL et al.: "Immune complexes in the acquired immunodeficiency syndrome: Relationship to desease manifestion, risk group and immunologic defect", s. abstract 13172, & J. Clin. Immunol. 5(2): 130-138, 1985
- Biological Abstracts, vol. 79, March 1985, (Philadelphia, PA, US) M.A. Cavaille-Coll et al.: "Critical analysis of T cell subset and function evaluation in patients with persistent generalized lymphadenopathy in groups at risk for acquired immune deficiency syndrome", s. abstract 22340, & Clin. Exp. Immunol. 57(3): 511-519, 1984
- Biological Abstracts, vol. 81, February 1986, (Philadelphia, PA, US)J. Taylor et al.: "Prognostically significant classification of immune changes in acquired immunodeficiency syndrome with Kaposi's sarcoma", s. abstract 112004, & Blood 67(3), 666-671, 1986
- Journal of Immunological Methods, vol. 91, no. 2, 1986, (Amsterdam, NL) S. Chollet-Martin et al.: "Enumeration of the T4 positive and T8 positive lymhocytes in AIDS and related syndromes", pp. 271-274, s. "Introduction"
- Biological Abstracts, vol. 83, September 1987, (Philadelphia, PA, US) R. Afrasiabi et al.: Characterization of a distinct subgroup of high-risk persons with Karoposi's sarcoma and good prognosis who present with normal T4 cell number and T4:T8 ratio and negative HTLV-III/LAV biologic test results", s. abstract 87953, & AM. J. MED 81(6): 969-973, 1986

## Description

Primary immunodeficiency is a very rare, but devastating, condition in which there is a partial or total collapse or absence of one or more classes of immune responses. It is typified by recurrent or chronic life-threatening infections and by the necessity for its victims to live in rigorously controlled, sterile conditions. There is no existing treatment other than isolation of the patient from all potential sources of infection.

In secondary immunodeficiency, the immune system function is reduced and may appear as a result of age, disease or the use of certain therapies, such as cytostatic radiotherapy or chemotherapy for cancer. Patients whose immune system function is reduced are vulnerable to both viral and other infections, and treatment of such infections may be complicated and/or protracted.

Acquired Immune Deficiency Syndrome (AIDS) is a recently identified syndrome in which a person's immune system after functioning normally ceases to function adequately.

The etiology of Acquired Immune Deficiency Syndrome has not yet been unequivocally established, although HTLV-III virus is believed to be implicated, and no effective treatment has yet been found. Because of the breakdown in the immune system, such individuals are highly vulnerable to infections and there has been a high mortality from opportunistic infections and Kaposi's sarcoma. See, e.g. "Immunocompromised Homosexuals" (Editorial) Lancet 1981, ii: 1325-6; Center for Disease Control, "Epidemiological Aspects of the Current Outbreak of Kaposi's Sarcoma and Opportunistic Infections", New England Journal of Medicine 1982, 306: 248-52; Gerstoft et al., "Severe Acquired Immunodeficiency in European Homosexual Men", British Medical Journal 1982, 285: 17-19. The two year mortality rate has been reported to be as high as 80%.

Lymphadenopathy Syndrome (LAS) is also believed to be caused by the HTLV-III virus. For reasons which are unknown, some Lymphadenopathy Syndrome patients develop AIDS while others do not. A diagnostic method of forecasting whether an individual suffering from Lymphadenopathy Syndrome will develop or will not develop AIDS is, therefore, quite valuable.

One of the characteristics of both Lymphadenopathy Syndrome and Acquired Immune Deficiency Syndrome is a decrease in the number of T helper cells defined phenotypically by T4 or Leu 3 monoclonal antibodies.

In Nicholson et al., "Alterations of Functional Subsets of T Helper and T Suppressor Cell Populations in Acquired Immunodeficiency Syndrome (AIDS) and Chronic Unexplained Lymphadenopathy", Journal of Clinical Immunology, Vol. 5: 267-274 (1985), a study of the distribution of Leu 8 and Ia on T cells of the helper (T4) and suppressor (T8) phenotype in homosexual men with chronic unexplained lymphadenopathy, homosexual men with Acquired Immune Deficiency Syndrome, and homosexual and heterosexual controls is described. The authors determined that there was a decrease in the T4:Leu 8⁺ subset of helper T cells in both conditions. However, in the AIDS patients, the T4:Leu 8⁻ subset was decreased while in the lymphadenopathy patients, it was not. While potentially useful in the basic research which is being carried out, the measurement of particular functional subsets of T cells is, to the treating physician, most inconvenient.

In "Scientific American", January 1986, pages 70-79, it is disclosed that the Aids virus alters the growth and function of T4 lymphocytes, a class of white blood cells that is crucial to the immune system.

It has now been determined that the degree of T4 suppression in lymphadenopathy patients provides a reasonable predictive factor to assess which of the individuals will develop Acquired Immune Deficiency Syndrome, without having to analyze T4 subsets which is both inconvenient and time consuming. While all of the ARC patients have reduced T4 populations, it has been noted that those who develop Acquired Immune Deficiency Syndrome have a significantly lover T4 count than those who do not. It now appears that a T4 lymphocyte count in the neighborhood of 300 cells/mm³ divides the individuals into those who are likely to develop Acquired Immune Deficiency Syndrome and those who are not. A statistically significant (i.e. p < 0.05) drop in the T4 count upon serial, time spaced assays is also indicative of those LAS individuals who will probably develop AIDS.

Serial assays can be conducted at fixed time intervals, e.g., weekly, monthly or bimonthly, or in combinations of such intervals. Preferably, assays will be conducted at least once a month.

Numerous in vitro assays for the measurement of T4 are known and any of these can be employed. Many of these assays are commercially available. OK-T monoclonal antibody-based assays, such as those manufactured by Ortho Pharmaceutical Co., are preferred.

As part of a double blind study, nineteen homosexual men who had lymphadenopathy syndrome and who also had oral thrush, persistent diarrhea, with loss in excess of ten percent of body weight and/or persistent unexplained fever were prospectively evaluated. All of these individuals showed antibodies to HTLV-III in Elisa and Western Blot analytical procedures.

Seven of the 19 individuals who had ARC developed Acquired Immune Deficiency Syndrome over a period of two to eight months with a median development time of three months. Four of these patients developed Pneumocystis carnii pneumonia, one developed Kaposi's sarcoma, one developed disseminated Mycobacterium avium intracellulare and one developed disseminated cytomegalovirus infection. Three of the seven individuals died at two, three and eight months, respectively, during the course of this study. The T4 lymphocytes of these individuals were measured at the start of the study with a mean value of 131±145 cells/mm³. Six months or less into the study (depending on individual survival), this group had a mean T4 count of 158±158 cells/mm³. It was also noted that while the initial T4 lymphocyte counts for the members of this group were not significantly different, those living individuals with AIDS at the end of the study period had a significantly higher (p < 0.05) mean T4 lymphocyte count (235±143 cells/mm³) relative to those who had died (29±22 cells/mm³).

The twelve remaining individuals had lymphadenopathy syndrome and life table analysis revealed a 57% AIDS free survival at ten months, range 4-10 months. The initial mean T4 lymphocyte count of the individuals in this group was 335±275 cells/mm³ and about six months into the study, the mean T4 lymphocyte count was 257±230 cells/mm³. These results show that the initial T4 lymphocyte counts were significantly lower in the group which developed AIDS and the difference was statistically significant.

Various changes and modifications can be made in the method described above by the skilled artisan without departing from the spirit and scope of the invention.

## Claims

1. A method of assessing whether an individual having lymphadenopathy syndrome (LAS) will develop Acquired Immune Deficiency Syndrome (AIDS) which comprises conducting in vitro T4 diagnostic assays and determining whether the mean T4 lymphocyte count is less than 300 cells/mm³.

2. The method of claim 1 wherein a plurality of time spaced assays are conducted.

3. The method of claim 2 wherein the assays are conducted at least monthly.

## Patentansprüche

1. Verfahren zur Bewertung, ob ein Patient mit Lymphadenopathie-Syndrom (LAS) das erworbene Immunmangelsyndrom (AIDS) entwickelt, dadurch **gekennzeichnet,** daß man diagnostische in vitro T4-Assays durchführt und bestimmt, ob die mittlere T4-Lymphozytenzahl weniger als 300 Zellen/mm³ beträgt.

2. Verfahren nach Anspruch 1, dadurch **gekennzeichnet,** daß eine Vielzahl von Assays mit zeitlichem Abstand durchgeführt werden.

3. Verfahren nach Anspruch 2, dadurch **gekennzeichnet,** daß die Assays mindestens monatlich durchgeführt werden.

## Revendications

1. Procédé pour évaluer si un individu ayant un syndrome de lymphadénopathie (LAS) va développer un syndrome immuno-déficitaire acquis (SIDA), qui comprend la réalisation d'essais diagnostiques de T4 in vitro et la détermination du compte moyen de lymphocytes T4 pour savoir si celui-ci est inférieur à 300 cellules/mm³.

2. Procédé suivant la revendication 1, dans lequel de nombreux essais espacés dans le temps sont effectués.

3. Procédé suivant la revendication 2, dans lequel les essais sont effectués au moins tous les mois.
